# EUROPÄISCHE PATENTSCHRIFT

(11) **EP 1 789 415 B1**
(45) Veröffentlichungstag und Bekanntmachung des Hinweises auf die Patenterteilung: **01.12.2010**
(21) Anmeldenummer: 05772974.1
(22) Anmeldetag: 04.08.2005
(51) Int. Cl.: C07D 471/06, C09B 5/62

(54) **VERFAHREN ZUR HERSTELLUNG VON QUATERRYLEN-3,4:13,14-TETRACARBONSÄUREDIIMIDEN DURCH DIREKTSYNTHESE**
METHOD FOR PRODUCING QUATERRYLENE-3,4:13,14-TETRACARBOXY DIIMIDES BY DIRECT SYNTHESIS
PROCEDE DE PRODUCTION DE DIIMIDES DE QUATERRYLENE-3,4:13,14-ACIDE TETRACARBOXYLIQUE, PAR SYNTHESE DIRECTE

(30) Priorität: 26.08.2004 DE 102004041604
(43) Veröffentlichungstag der Anmeldung: 30.05.2007
(73) Patentinhaber: BASF SE, 67056 Ludwigshafen (DE)
(72) Erfinder: KÖNEMANN, Martin, 68163 Mannheim (DE); BÖHM, Arno, 40764 Langenfeld (DE); BIDLINGMAIER, Hermann, 74343 Sachsenheim (DE); RIEGER, Reinhold, 67165 Waldsee (DE); BLASCHKA, Peter, 67069 Ludwigshafen (DE); REICHELT, Helmut, 67435 Neustadt (DE); KRIEGER, Matthias, CH-4058 Basel (CH)
(86) Internationale Anmeldenummer: PCT/EP2005/008443
(87) Internationale Veröffentlichungsnummer: WO 2006/021307

(56) Entgegenhaltungen:
- EP-A- 0 596 292
- DE-A1- 10 233 955
- DE-A1- 19 512 773
- LANGHALS H ET AL: "A Two-Step Synthesis of Quaterrylenetetracarboxylic Bisimides-Novel NIR Fluorescent Dyes" TETRAHEDRON LETTERS, ELSEVIER, AMSTERDAM, NL, Bd. 36, Nr. 36, 4. September 1995 (1995-09-04), Seiten 6423-6424, XP004027248 ISSN: 0040-4039 in der Anmeldung erwähnt

## Beschreibung

Die vorliegende Erfindung betrifft ein neues Verfahren zur Herstellung von Quaterrylen-3,4:13,14-tetracarbonsäurediimiden der allgemeinen Formel I 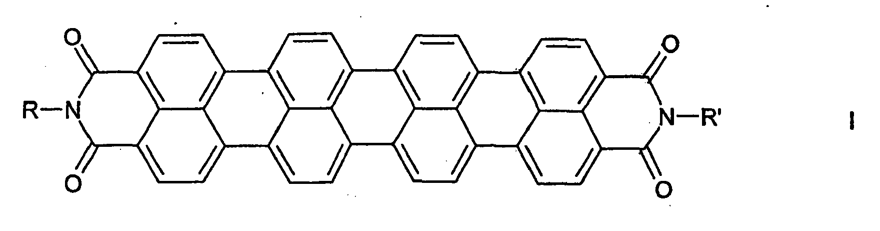 in der die Variablen folgende Bedeutung haben:
- R, R': unabhängig voneinander:
Wasserstoff;
   (A) C₁-C₃₀-Alkyl, dessen Kohlenstoffkette durch eine oder mehrere Gruppie- rungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch:
      (i) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
      (ii) Aryl oder Hetaryl, an das weitere gesättigte oder ungesättigte 5- bis 7- gliedrige Ringe, deren Kohlenstoffgerüst durch eine oder mehrere Gruppie- rungen -O-, -S-, -NR¹-, -N=CR¹-, -CR¹=CR¹-, -CO-, -SO- und/oder -SO₂- un- terbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³ substituiert sein kann;
      (iii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁- C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
      (iv) einen Rest -U-Aryl, der ein- oder mehrfach durch die vorstehenden, als Substituenten für die Arylreste (ii) genannten Reste substituiert sein kann, wo- bei U eine Gruppierung -O-, -S-, -NR¹-, -CO-, -SO- oder -SO₂- bedeutet;
   (B) C₃-C₈-Cycloalkyl, an das weitere gesättigte oder ungesättigte 5- bis 7- gliedrige Ringe, deren Kohlenstoffgerüst durch eine oder mehrere Gruppie- rungen -O-, -S-, -NR¹-, -N=CR¹-, -CR¹=CR¹-, -CO-, -SO- und/oder -SO₂- un- terbrochen sein kann, anneliert sein können, wobei das gesamte Ringsystem ein- oder mehrfach sein kann durch die Reste (i), (ii), (iii), (iv) und/oder (v) C₁-C₃₀-Alkyl, dessen Kohlenstoffkette durch eine oder mehrere Gruppie- rungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ ,-POR²R³, Aryl und/oder gesättigtes oder ungesättig- tes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituen- ten für Alkyl genannten Reste substituiert sein können;
   (C) Aryl oder Hetaryl, an das weitere gesättigte oder ungesättigte 5- bis 7- gliedrige Ringe, deren Kohlenstoffgerüst durch eine oder mehrere Gruppie- rungen -O-, -S-, -NR¹-, -N=CR¹-, -C≡C-, -CR¹=CR¹-, -CO-, -SO- und/oder -SO₂- unterbrochen sein kann, anneliert sein können, wobei das gesamte Ringsystem substituiert sein kann durch die Reste (i), (ii), (iii), (iv), (v) und/oder Aryl- und/oder Hetarylazo, das jeweils durch C₁-C₁₀-Alkyl, C₁-C₆-Alkoxy und/oder Cyano substituiert sein kann;
- R¹: Wasserstoff oder C₁-C₁₈-Alkyl, wobei die Reste R¹ gleich oder verschieden sein können, wenn sie mehrfach auftreten;
- R², R³: unabhängig voneinander Wasserstoff;
C₁-C₁₈-Alkyl, dessen Kohlenstoffkette durch eine oder mehrere Gruppierun- gen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halo- gen, Cyano, Nitro und/oder -COOR¹ substituiert sein kann;
Aryl oder Hetaryl, an das jeweils weitere gesättigte oder ungesättigte 5- bis 7- gliedrige Ringe, deren Kohlenstoffgerüst durch eine oder mehrere Gruppie- rungen -O-, -S-, -CO- und/oder -SO₂- unterbrochen sein kann, anneliert sein können, wobei das gesamte Ringsystem ein- oder mehrfach durch C₁-C₁₂- Alkyl und/oder die vorstehenden, als Substituenten für Alkyl genannten Reste substituiert sein kann.

Quaterrylen-3,4:13,14-tetracarbonsäurediimide sind bekanntermaßen von besonderem Interesse als Pigmente und Nah-IR-Absorber.

In der EP-A-596 292 bzw. Angew. Chem. 107, S. 1487-1489 (1995) ist erstmals die Synthese von N,N'-didodecyl- und N,N'-bis(2,6-diisopropylphenyl)substituiertem Quaterrylen-3,4:13,14-tetracarbonsäurediimid beschrieben. Hierbei wird das entsprechend am Imidstickstoffatom substituierte 9-Bromperylen-3,4-dicarbonsäureimid unter Bromabspaltung in Gegenwart eines inerten Verdünnungsmittels (Dimethylformamid) und eines übergangsmetallorganischen Katalysators (Bis(1,5-Cyclooctadien)nickel) zum Biperylenderivat umgesetzt, das schließlich durch Erhitzen in alkalischem Medium in Gegenwart eines Oxidationsmittels in das Quaterrylenderivat überführt wird. Bei diesem Verfahren ist nachteilig, daß es auf die Herstellung bestimmter Quaterrylentetracarbonsäurediimide beschränkt ist und die Produkte insbesondere für den Einsatz in Kunststoffen unerwünschte Katalysatorspuren enthalten.

In der DE-A-102 33 955 wird die Herstellung von N,N'-Bis(1-hexylheptyl)quaterrylen-3,4:13,14-tetracarbonsäurediimid durch ein ebenfalls zweistufiges Verfahren in Gegenwart unerwünschter Schwermetallkatalysatoren beschrieben, in dessen ersten Schritt N-(1-Hexylheptyl)perylen-3,4-anhydrid-9,10-imid in Gegenwart von Kupferpulver zum Biperylenderivat umgesetzt wird, das im zweiten Schritt zum Quaterrylenderivat oxidiert wird.

Aus Tetrahedron Letters, 36, S. 6423-6424 (1995) ist die Herstellung von unsubstituiertem und N,N'-Bis(1-hexylheptyl)quaterrylen-3,4:13,14-tetracarbonsäurediimid durch Erhitzen des entsprechenden Perylen-3,4-dicarbonsäureimids in 85 gew.-%iger Kalilauge auf 290-300°C bekannt. Derartig aggressive Reaktionsbedingungen stellen jedoch extreme Anforderungen an die verwendeten Apparaturen, zudem liegt die Ausbeute nur bei 4%.

Schließlich wird in J. Org. Chem. 69, S. 2719-2726 (2004) N,N'-Bis(2-Diethylaminoethyl)quaterrylen-3,4:13,14-tetracarbonsäurediimid als Vorstufe für das entsprechende flüssigkristalline, ionisch als Acetat vorliegende Diimid beschrieben. Seine Herstellung erfolgt durch Homokupplung des über mehrere Reaktionsstufen hergestellten N-(2-Diethylaminoethyl)-9-bromperylen-3,4-dicarbonsäureimids in Gegenwart von Nickelchlorid, Triphenylphosphin und Dimethylformamid. Auch bei diesem Spezialverfahren werden unerwünschte toxische Übergangsmetallkatalysatoren eingesetzt.

Der Erfindung lag daher die Aufgabe zugrunde, diesen Nachteilen abzuhelfen und ein Verfahren bereitzustellen, das die Herstellung von Quaterrylen-3,4:13,14-tetracarbonsäurediimiden auf vorteilhafte, wirtschaftliche Weise ermöglicht.

Demgemäß wurde ein Verfahren zur Herstellung von Quaterrylen-3,4:13,14-tetracarbonsäurediimiden der allgemeinen Formel I 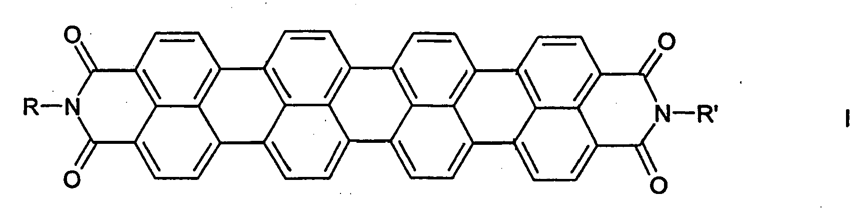 in der die Variablen die eingangs angegebene Bedeutung haben, gefunden, welches dadurch gekennzeichnet ist, daß man ein Perylen-3,4-dicarbonsäureimid der allgemeinen Formel IIa 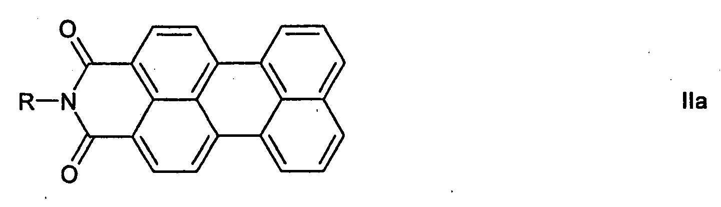 in Gegenwart eines basenstabilen, hochsiedenden, organischen Lösungsmittels und einer alkali- oder erdalkalimetallhaltigen Base mit einem Perylen-3,4-dicarbonsäureimid der allgemeinen Formel IIb 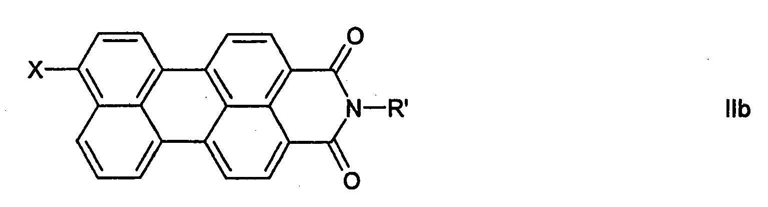 in der X Wasserstoff, Brom oder Chlor bedeutet, umsetzt.

Bevorzugte Anwendung findet das erfindungsgemäße Verfahren zur Herstellung von Quaterrylen-3,4:13,14-tetracarbon-säurediimiden der Formel I, in der die Variablen folgende Bedeutung haben:
- R, R': unabhängig voneinander Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substitu- iert sein kann, und/oder einen über ein Stickstoffatom gebundenen 5- bis 7- gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppie- rungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, Halogen, -CONHR² und/oder Aryl- oder Hetarylazo, das jeweils durch C₁-C₁₀-Alkyl, C₁- C₆-Alkoxy oder Cyano substituiert sein kann, ein- oder mehrfach substituiert sein kann;
- R¹: Wasserstoff oder C₁-C₆-Alkyl;
- R²: Wasserstoff; C₁-C₁₈-Alkyl; Aryl oder Hetaryl, das jeweils durch C₁-C₆-Alkyl, C₁- C₆-Alkoxy, Halogen, Hydroxy, Carboxy oder Cyano substituiert sein kann.

Alle in den Formeln I und II auftretenden Alkylgruppen können geradkettig oder verzweigt sein. Wenn die Alkylgruppen substituiert sind, tragen sie in der Regel 1 oder 2 Substituenten.

Cycloalkylgruppen und aromatische Reste, die substituiert sind, können im allgemeinen bis zu 3, bevorzugt 1 oder 2, der genannten Substituenten aufweisen.

Als Beispiele für geeignete Reste R, R', R¹ und R² (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
Methoxymethyl, 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
Methylthiomethyl, 2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulf oxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsulfoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-lsopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
Cyanomethyl, 2-Cyanoethyl, 3-Cyanopropyl, 2-Methyl-3-ethyl-3-cyanopropyl, 7-Cyano-7-ethylheptyl und 4,7-Dimethyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Ethinyl, 1- und 2-Propinyl, 1-, 2- und 3-Butinyl, 1-, 2-, 3- und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl;
Ethenyl, 1- und 2-Propenyl, 1-, 2- und 3-Butenyl, 1-, 2-, 3- und 4-Pentenyl, 1-, 2-, 3-, 4- und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16- und 17-Octadecenyl;
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-N-ethylaminosulfonyl, N-Methyl-N-dodecylaminosulfonyl, N-Dodecylaminosulfonyl, (N,N-Dimethylamino)ethylaminosulfonyl, N,N-(Propoxyethyl)dodecylaminosulfonyl, N,N-Diphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butylphenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Chlor, Brom und Iod;
Phenylazo, 2-Napthylazo, 2-Pyridylazo und 2-Pyrimidylazo;
Phenyl, 1- und 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5- Isochinolyl;
2-, 3- und 4-Methylphenyl, 2,3-, 2,4-, 2,5-, 3,5- und 2,6-Dimethylphenyl, 2,4,6-Trimethylphenyl, 2-, 3- und 4-Ethylphenyl, 2,3-, 2,4-, 2,5-, 3,5- und 2,6-Diethylphenyl, 2,4,6-Triethylphenyl, 2-, 3- und 4-Propylphenyl, 2,3-, 2,4-, 2,5-, 3,5- und 2,6-Dipropylphenyl, 2,4,6-Tripropylphenyl, 2-, 3- und 4-Isopropylphenyl, 2,3-, 2,4-, 2,5-, 3,5- und 2,6-Diisopropylphenyl, 2,4,6-Triisopropylphenyl, 2-, 3- und 4-Butylphenyl, 2,3-, 2,4-, 2,5-, 3,5- und 2,6-Dibutylphenyl, 2,4,6-Tributylphenyl, 2-, 3- und 4-Isobutylphenyl, 2,3-, 2,4-, 2,5-, 3,5- und 2,6-Diisobutylphenyl, 2,4,6-Triisobutylphenyl, 2-, 3- und 4-sec.-Butylphenyl, 2,3-, 2,4-, 2,5-, 3,5- und 2,6-Di-sec.-butylphenyl und 2,4,6-Tri-sec.-butylphenyl, 2-, 3- und 4-tert.-Butylphenyl, 2,3-, 2,4-, 2,5-, 3,5- und 2,6-Di-tert.-butylphenyl, 2,4,6-Tri-tert.-butylphenyl; 2-, 3- und 4-Methoxyphenyl, 2,3-, 2,4-, 2,5-, 3,5- und 2,6-Dimethoxyphenyl, 2,4,6-Trimethoxyphenyl, 2-, 3- und 4-Ethoxyphenyl, 2,3-, 2,4-, 2,5-, 3,5- und 2,6-Diethoxyphenyl, 2,4,6-Triethoxyphenyl, 2-, 3- und 4-Propoxyphenyl, 2,3-, 2,4-, 2,5-, 3,5- und 2,6-Dipropoxyphenyl, 2-, 3- und 4-Isopropoxyphenyl, 2,3-, 2,4-, 2,5-, 3,5- und 2,6-Diisopropoxyphenyl und 2-, 3- und 4-Butoxyphenyl; 2-, 3- und 4-Chlorphenyl, und 2,3-, 2,4-, 2,5-, 3,5- und 2,6-Dichlorphenyl; 2-, 3- und 4-Hydroxyphenyl und 2,3-, 2,4-, 2,5-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3-und 4-N-(o-Tolyl)aminophenyl, 3- und 4-N-(m-Tolyl)aminophenyl und 3- und 4-N-(p-Tolyl)aminophenyl; 3- und 4-(2-Pyridyl)aminophenyl, 3- und 4-(3-Pyridyl)aminophenyl, 3-und 4-(4-Pyridyl)aminophenyl, 3- und 4-(2-Pyrimidyl)aminophenyl und 4-(4-Pyrimidyl)-aminophenyl;
4-Phenylazophenyl, 4-(1-Naphthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Cyclopropyl, Cyclobutyl. Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4-Nitrocyclohexyl und 3- und 4-Chlorcyclohexyl;
1-, 2- und 3-Cyclopentenyl, 1-, 2-, 3- und 4-Cyclohexenyt, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl.

Mit Hilfe des erfindungsgemäßen Verfahrens können die Quaterrylen-3,4:13,14-tetracarbonsäurediimide I in einem Schritt durch Umsetzung eines Perylen-3,4-dicarbonsäureimids IIa (im folgenden Imid IIa genannt) mit einem Perylen-3,4-dicarbonsäureimid IIb (im folgenden Imid IIb genannt) in Gegenwart eines basenstabilen, hochsiedenden, organischen Lösungsmittels und einer alkali- oder erdalkalimetallhaltigen Base hergestellt werden.

Als Imid IIb kann dabei sowohl ein in 9-Position halogeniertes, also chloriertes oder insbesondere bromiertes, als auch ein nichthalogeniertes Imid, das am Imidstickstoffatom einen Rest R' tragen kann, der mit dem Rest R am Imidstickstoffatom des Imids IIa übereinstimmt oder von diesem verschieden ist, eingesetzt werden.

Der Einsatz von halogeniertem Imid IIb ermöglicht dabei die gezielte Synthese unsymmetrischer Quaterrylen-3,4:13,14-tetracarbonsäurediimide I (R ≠ R'). In diesem Fall ist es vorteilhaft, ein Molverhältnis IIb zu IIa von 4 : 1 bis 1 : 1, insbesondere von 2 : 1 bis 1 : 1, zu verwenden.

Wird nichthalogeniertes Imid IIb verwendet, so empfiehlt es sich in der Regel, die Umsetzung bei verschärften Reaktionsbedingungen vorzunehmen, d.h. zusätzlich zu einer starken alkallmetallhaltigen Base eine stickstoffhaltige Hilfsbase einzusetzen.

Als Lösungsmittel sind grundsätzlich alle unter den Reaktionsbedingungen gegen Basen stabilen, hochsiedenden Lösungsmittel (Siedepunkt > 100°C und oberhalb der gewählten Reaktionstemperatur) geeignet, in denen sich die verwendeten Basen bei Reaktionstemperatur vollständig und die Imide IIa und IIb zumindest partiell, bevorzugt vollständig lösen, so daß weitgehend homogene Reaktionsbedingungen vorliegen. Es können sowohl aprotische (unpolar-aprotische und polar-aprotische) als auch protische Lösungsmittel eingesetzt werden. Selbstverständlich können auch Lösungsmittelmischungen verwendet werden.

Beispiele für geeignete unpolar-aprotische Lösungsmittel sind bei > 100°C siedende Kohlenwasserstoffe aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cydoalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für bevorzugte unpolar-aprotische Lösungsmittel seien im einzelnen genannt:
Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan;
Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin, 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ, und Alkylbenzolgemische vom Solvesso^{®} Typ.

Besonders bevorzugte unpolar-aprotische Lösungsmittel sind Xylol (alle Isomere), Mesitylen und vor allem Dekalin.

Beispiele für geeignete polar-aprotische Lösungsmittel sind stickstoffhaltige Heterocyclen und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für bevorzugte polar-aprotische Lösungsmittel seien im einzelnen genannt:
Chinolin, Isochinolin, Chinaldin, Pyrimidin, N-Methylpiperidin und Pyridin;
Dimethyl- und Tetramethyltetrahydrofuran und Dioxan;
Diphenylether; Ethylenglykoldiethyl-, -dipropyl-, -diisopropyl-, -di-n-butyl-, -di-sec.-butyl- und -di-tert.-butylether und Ethylenglykolmethylethylether, Di- und Triethylenglykoldimethyl-, -diethyl-, -dipropyl-, -diisopropyl-, -di-n-butyl-, -di-sec.-butyl- und -di-tert.-butylether und Di- und Triethylenglykolmethylethylether.
Dabei sind Diethylenglykoldiethylether, Diphenylether und vor allem Diethylenglykoldimethylether besonders bevorzugt.
Beispiele für geeignete protische Lösungsmittel sind bei > 100°C siedende einwertige und mehrwertige, aliphatische und aromatische Alkohole (insbesondere einwertige C₄-C₁₈-Alkanole, mehrwertige C₂-C₄-Alkohole und deren Oligomere, wie C₂-C₃-Alkylenglykole, die bis zu 6 Alkylenoxideinheiten enthalten können, und Phenole), Etheralkohole (insbesondere Mono-C₁-C₆-alkyl- und -phenylether von monomeren und oligomeren C₂-C₃-Alkylenglykolen, die bis zu 6 Alkylenoxideinheiten enthalten können, vor allem Ethylenglykolmono-C₄-C₆-alkylether) und Aminoalkohole (insbesondere Mono-, Di- und Tri-C₂-C₄-alkoholamine).

Als Beispiele für bevorzugte protische Lösungsmittel seien im einzelnen genannt:
n-Butanol, Isobutanol, tert.-Butanol, n-Pentanol, Isopentanol, 2-Methylbutanol, 2-Methyl-2-butanol (tert.-Amylalkohol), Hexanol, 2-Methylpentanol, 3-Methyl-3-pentanol, Heptanol, 1-Ethylpentanol, 3-Ethyl-3-pentanol, 2,3-Dimethyl-3-pentanol, Octanol, 2-Ethylhexanol, 2,4,4-Trimethyl-2-pentanol, Isooctylakohol, Nonanol, Isononylalkohol; Decanol, 2,2,3,4,4-Pentamethyl-3-pentanol, Isodecylalkohol, Undecanol, Dodecanol, Tridecanol, Isotridecylalkohol, Tetradecanol, Pentadecanol, Hexadecanol, Heptadecanol und Octadecanol;
Ethylenglykol, Diethylenglykol, Triethylenglykol, Tetraethylenglykol, Pentaethylenglykol und Hexaethylenglykol, Propylenglykol, 1,3-Propandiol, Glycerin und 1,2-, 1,3- und 1,4-Butandiol;
Ethylenglykolmonomethyl-, -ethyl-, -propyl-, -isopropyl-, -n-butyl-, -sec.-butyl-, -tert.-butyl-, -n-pentyl- und -n-hexylether und Ethylenglykolmonophenylether und Di- und Triethylenglykolmonomethyl-, -ethyl-, -propyl-, -isopropyl-, -n-butyl-, -sec.-butyl-, -tert.-butyl-, -n-pentyl- und -n-hexylether und Di- und Triethylenglykolmonophenylether;
Monoethanolamin, Diethanolamin und Triethanolamin.

Besonders bevorzugte protische Lösungsmittel sind Ethylenglykol und Ethanolamin.

Die Lösungsmittelmenge beträgt in der Regel 1 bis 20 g, bevorzugt 2 bis 10 g und besonders bevorzugt 2 bis 5 g je g Imid IIa und IIb.

Als Base sind starke anorganische und organische alkali- oder erdalkalimetallhaltige Basen geeignet, wobei die alkalimetallhaltigen Basen besonders geeignet sind. Bevorzugte anorganische Basen sind Alkali- und Erdalkalimetallhydroxide und -amide, bevorzugte organische Basen sind Alkali- und Erdalkalimetallalkoholate (insbesondere die C₁-C₁₀-Alkoholate, vor allem tert.-C₄-C₁₀-Alkoholate), Alkali- und Erdalkalimetall-(phenyl)alkylamide (insbesondere die Bis(C₁-C₄-alkyl)amide) und Triphenylmethylmetallate. Besonders bevorzugt sind die Alkalimetallalkoholate. Bevorzugte Alkalimetalle sind Lithium, Natrium und Kalium, wobei Kalium ganz besonders bevorzugt ist. Besonders geeignete Erdalkalimetalle sind Magnesium und Calcium.

Als Beispiele für besonders bevorzugte Basen seien im einzelnen genannt: Lithiumhydroxid, Natriumhydroxid und Kaliumhydroxid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat und Kalium-tert.-butylat; Lithium-(1,1-dimethyl)octylact, Natrium-(1,1-dimethyl)octylat, Kalium-(1,1-dimethyl)octylat, Lithiumdimethylamid, Lithiumdiethylamid, Lithiumdiisopropylamid, Natriumdiisopropylamid, Triphenylmethyllithium, Triphenylmethylnatrium und Triphenylmethylkalium.

Ganz besonders bevorzugte Basen sind Lithiumdiisopropylamid, Natriummethylat, Natrium-tert.-butylat, vor allem Kaliummethylat und Kaliumhydroxid und insbesondere Kalium-tert.-butylat.

Bei Verwendung der Methylate und der Hydroxide sowie generell bei Verwendung von nichthalogenierten Imiden IIb empfiehlt sich zur Erhöhung der Reaktivitätder Zusatz einer stickstoffhaltigen Hilfsbase mit geringer nukleophiler Wirkung, wenn nicht bereits ein stickstoffhaltiger Heterocyclus oder ein Alkoholamin als Lösungsmittel anwesend ist. Geeignete Basen sind bei den Reaktionstemperaturen flüssige Alkylamine, insbesondere Tri-C₃-C₆-alkylamine, wie Tripropylamin und Tributylamin, Alkoholamine, insbesondere Mono-, Di- und Tri-C₂-C₄-alkoholamine, wie Mono-, Di- und Triethanolamin, und insbesondere heterocyclische Basen, wie Pyridin, N-Methylpiperidin, N-Methylpiperidon, N-Methylmorpholin, N-Methyl-2-pyrrolidon, Pyrimidin, Chinolin, Isochinolin, Chinaldin und vor allem Diazabicyclononen (DBN) und Diazabicycloundecen (DBU).

Geeignete Einsatzmengen für die Hilfsbase liegen im allgemeinen bei 0,5 bis 25 g, bevorzugt bei 1 bis 10 g, besonders bevorzugt bei 1 bis 3 g, je g Imid IIa und IIb.

Die Alkali- oder Erdalkalimetallbase wird in der Regel in Mengen von 2 bis 20 mol, insbesondere 2 bis 10 mol, je mol Imid IIa und IIb eingesetzt.

Die Alkalimetallbase kann in fester oder in gelöster Form eingesetzt werden. Wenn die Alkalimetallbase in Kombination mit einem unpolar-aprotischen Reaktionslösungsmittel verwendet wird, in dem sie nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Reaktionstemperatur liegt üblicherweise bei 70 bis 210°C, bevorzugt bei 120 bis 180°C.

Insbesondere bei Abwesenheit eine Hilfsbase kann es zur Herstellung unsymmetrischer Quaterrylen-3,4:13,14-tetracarbonsäurediimide vorteilhaft sein, zunächst eine Reaktionstemperatur im oberen Bereich zu wählen, um das Imid IIa in 9-Stellung zu deprotonieren. Die anschließende Kupplungsreaktion mit dem halogenierten Imid IIb kann dann in der Regel bei niedrigerer Temperatur durchgeführt werden, was sich insbesondere bei der Anwesenheit von basenlabilen Substituenten (z.B. Cyclohexyl) am Imidstickstoffatom empfiehlt.

Die Reaktionszeit beträgt in der Regel 1 bis 3 h bei Einsatz halogenierter Imide IIb und 2 bis 12 h bei Einsatz nichthalogenierter Imide IIb.

Verfahrenstechnisch geht man beim Einsatz nichthalogenierter Imide IIb, also insbesondere einer Homokondensation, zweckmäßigerweise wie folgt vor:
Man erhitzt Lösungsmittel, Base und gegebenenfalls Hilfsbase zur Homogenisierung unter Schutzgas und gibt Imid IIa und Imid IIb gegebenenfalls nach vorherigem Abkühlen unter Schutzgas zu und erhitzt die Mischung die gewünschte Zeit unter Rühren und unter Schutzgas auf die gewünschte Reaktionstemperatur. Nach Abkühlen auf Raumtemperatur fällt man die Quaterrylen-3,4:13,14-tetracarbonsäurediimide I durch Zugabe von einem protischen Lösungsmittel, das die anderen Komponenten löst, z.B. von C₁-C₆-Alkoholen oder Wasser, aus. Man filtriert ab und wäscht mit einem der genannten Lösungsmittel, insbesondere mit einem der Alkohole.

Bei Verwendung halogenierter Imide IIb kann man verfahrenstechnisch wie bei Verwendung nichthalogenierter Imide IIb vorgehen. Man kann jedoch auch zunächst nur ein Gemisch von Imid IIa, Base, gegebenenfalls Hilfsbase sowie Lösungsmittel unter Rühren und Schutzgas auf eine Temperatur im Bereich von 120 bis 210°C erhitzen (Deprotonierung) und das Imid IIb anschließend, gegebenenfalls nach Absenken der Temperatur auf 50 bis 120°C, zugeben.

Gelegentlich kann es zweckmäßig sein, das Reaktionsprodukt einer Oxidation zu unterziehen. Dies kann am einfachsten durch Einblasen von Luftsauerstoff in die noch warme Reaktionsmischung geschehen. Es ist jedoch auch möglich, Oxidationsmittel, wie vorzugsweise Wasserstoffperoxid, aber auch aldehydgruppenhaltige Zucker, z.B. Glukose, insbesondere nach der Reaktion zuzugeben.

Zur weiteren Reinigung kann man die Produkte I z.B. aus einem Gemisch von halogenierten Lösungsmitteln, wie Chloroform und Methylenchlorid, und Alkoholen, wie Methanol, Ethanol und Isopropanol, umkristallisieren. Alternativ kann man auch eine Säulenchromatographie an Kieselgel unter Verwendung von Methylenchlorid oder Aceton als Eluens vornehmen.

Eine weitere Reinigungsmethode besteht darin, die Produkte I aus N,N-disubstituierten aliphatischen Carbonsäureamiden, wie N,N-Dimethylformamid und N,N-Dimethylacetamid, oder stickstoffhaltigen Heterocyclen, wie N-Methylpyrrolidon, oder deren Gemischen mit Alkoholen, wie Methanol, Ethanol und Isopropanol, umzukristallisieren oder mit diesen Lösungsmitteln zu waschen.

Schließlich können die Produkte I auch aus Schwefelsäure fraktioniert werden.

Mit Hilfe des erfindungsgemäßen Verfahrens können die Quaterrylen-3,4:13,14-tetracarbonsäurediimide I in guten Ausbeuten (in der Regel von 30 bis 60%) und hohen Reinheiten (üblicherweise 90 bis 99%) auf wirtschaftliche Weise in einem Schritt hergestellt werden. Sowohl an den Imidstickstoffatomen symmetrisch als auch unsymmetrisch substituierte Quaterrylen-3,4:13,14-tetracarbonsäurediimide I sind auf vorteilhafte Weise zugänglich.

### Beispiele

### Beispiel 1 bis 7

Eine Mischung aus y g des Lösungsmittels L, b g Kalium-tert.-butylat als Base und gegebenenfalls h g Diazabicyclononen (DBN) bzw. Diazabicycloundecen (DBU) als Hilfsbase wurde zur Homogenisierung unter Rühren in einer Stickstoffatmosphäre auf 100°C erhitzt. Nach Abkühlen auf Raumtermperatur wurden x g des Perylen-3,4-dicarbonsäureimids II (bzw. xₐ g des Imids IIa und x_{b} g des Imids IIb) zugegeben und das Gemisch unter Stickstoff auf T°C erhitzt.

Nach einer Nachrührzeit von t h bei T°C unter Stickstoff, Abkühlen auf Raumtemperatur und gegebenenfalls Zugabe von 300 ml Methanol zur vollständigen Ausfällung wurde der gebildete Niederschlag abfiltriert, nacheinander mit kaltem Lösungsmittel L, Methanol, 10 gew.-%iger Schwefelsäure und Wasser bis zum farblosen Ablauf gewaschen und bei 100°C im Vakuum getrocknet. Zur weiteren Reinigung wurde das Rohprodukt aus Beispiel 2 einer fraktionierten Kristallisation aus Schwefelsäure unterzogen. Das Rohprodukt aus Beispiel 3 wurde aus N-Methylpyrrolidon umkristallisiert.

Weitere Einzelheiten zu diesen Versuchen sowie deren Ergebnisse sind in der folgenden Tabelle zusammengestellt.

**Tabelle**

| Bsp | x [g] | Imid II | y [g] | Lösungsmittel L | h [g] | Hilfsbase | Base b [g] | t [h] | T [°C] | Ausbeute [g]/[%] | Reinheit [%] |
|---|---|---|---|---|---|---|---|---|---|---|---|
| 1 | 24 | IIa1* | 60 | Diethylenglykol-dimethylether | 25 | DBN | 20 | 8 | 130 | 12,9/50 | 93 |
| 2 | 24 | IIa | 100 | Ethylenglykol-monobutylether | 25 | DBN | 40 | 4 | 160 | 11,3/44 | 95 |
| 3 | 24 | IIa1 | 60 | Ethanolamin | 50 | DBU | 40 | 6 | 170 | 13,8/45 | 99 |
| 4 | 24 | IIa1 | 60 | Diethanolamin | 25 | DBN | 35 | 8 | 140 | 7,9/32 | 97 |
| 5 | 20 | IIa2** | 300 | Chinolin | - | - | 20 | 8 | 200 | 7,3 / 34 | 92 |
| 6 | 14 12 | IIa1 IIb*** | 60 | Diethylenglykol-dimethylether | 50 | DBU | 40 | 6 | 190 | 5,3/21 | 90 |

| | | | | | | | | | | | |
|---|---|---|---|---|---|---|---|---|---|---|---|
| * IIa1: N-(2,6-Diisopropylphenyl)perylen-3,4-dicarbonsäureimid ** IIa2: N-Phenylperylen-3,4-dicarbonsäureimid *** IIb: 9-Brom-N-(2,6-diisopropylphenyl)perylen-3,4-dicarbonsäureimid | | | | | | | | | | | |

## Patentansprüche

1. Verfahren zur Herstellung von Quaterrylen-3,4:13,14-tetracarbonsäurediimiden der allgemeinen Formel I 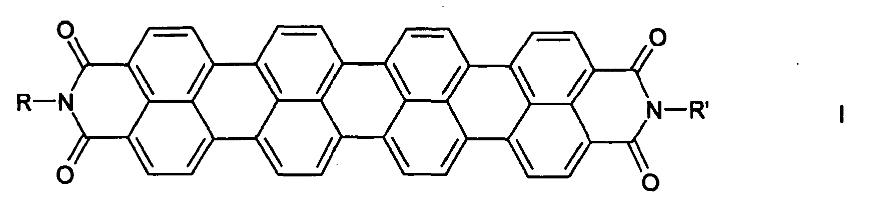 in der die Variablen folgende Bedeutung haben:
R, R' unabhängig voneinander:
Wasserstoff;
(A) C₁-C₃₀-Alkyl, dessen Kohlenstoffkette durch eine oder mehrere Grup-pierungen -O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch:
(i) C₁-C₁₂-Alkoxy, C₁-C₆-Alkylthio, -C≡CR¹, -CR¹=CR¹₂, Hydroxy, Mer- capto, Halogen, Cyano, Nitro, -NR²R³, -NR²COR³, -CONR²R³, -SO₂NR²R³, -COOR², -SO₃R², -PR²R³ und/oder -POR²R³;
(ii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₈-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydro- xy, Mercapto, Halogen, Cyano, Nitro, -NR²R³, -NR²COR³, -CONR²R³, -SO₂NR²R³, -COOR², -SO₃R², -PR²R³ und/oder -POR²R³ substituiert sein kann;
(iii) C₃-C₈-Cycloalkyl, dessen Kohlenstoffgerüst durch eine oder mehre- re Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anne- liert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder -POR²R³;
(iv) einen Rest -U-Aryl, der ein- oder mehrfach durch die vorstehenden, als Substituenten für die Arylreste (ii) genannten Reste substituiert sein kann, wobei U eine Gruppierung -O-, -S-, -NR¹-, -CO-, -SO- oder -SO₂- bedeutet;
(B) C₃-C₈-Cycloalkyl, an das weitere gesättigte oder ungesättigte 5- bis 7₋gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach sein kann durch: die Reste (i), (ii), (iii), (iv) und/oder (v) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach sub- stituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ ,-POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehr- fach durch C₁-C₁₈-Alkyl und/oder die vorstehenden, als Substituenten für Alkyl genannten Reste substituiert sein können;
(C) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -C≡C-, -CR¹=CR¹-, -CO-, -SO- und/oder -SO₂- unterbrochen sein kann, anneliert sein können, wobei das gesamte Ringsystem substituiert sein kann durch die Reste (i), (ii), (iii), (iv), (v) und/oder Aryl- und/oder Hetarylazo, das jeweils durch C₁- C₁₀-Alkyl, C₁-C₆-Alkoxy und/oder Cyano substituiert sein kann;
R¹ Wasserstoff oder C₁-C₁₈-Alkyl, wobei die Reste R¹ gleich oder verschie- den sein können, wenn sie mehrfach auftreten;
R²,R³ unabhängig voneinander Wasserstoff;
C₁-C₁₈-Alkyl, dessen Kohlenstoffkette durch eine oder mehrere Gruppie- rungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehr- fach durch C₁-C₁₂-Alkyl und/oder die vorstehenden, als Substituenten für Alkyl genannten Reste substituiert sein kann,
**dadurch gekennzeichnet, daß** man ein Perylen-3,4-dicarbonsäureimid der allgemeinen Formel IIa 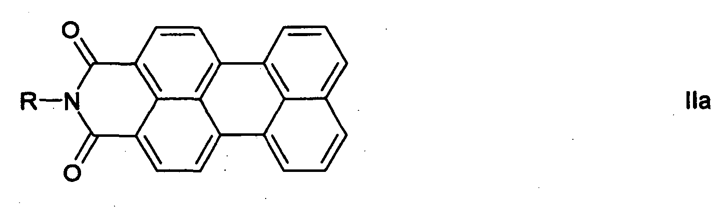 in Gegenwart eines basenstabilen, hochsiedenden, organischen Lösungsmittels und einer starken alkali- oder erdalkalimetallhaltigen Base bei einer Temperatur von 50 bis 210°C mit einem Perylen-3,4-dicarbonsäureimid der allgemeinen Formel IIb 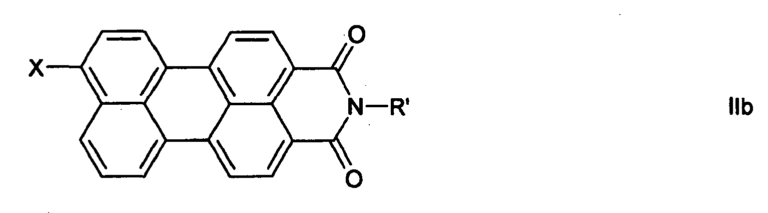 in der X Wasserstoff, Brom oder Chlor bedeutet, umsetzt.

2. Verfahren nach Anspruch 1, **dadurch gekennzeichnet, daß** man es zur Herstellung von Quaterrylen-3,4:13,14-tetracarbonsäurediimiden der Formel verwendet, in der die Variablen folgende Bedeutung haben:
R, R' unabhängig voneinander:
Wasserstoff;
C₁-C₃₀-Alkyl, dessen Kohlenstoffkette durch eine oder mehrere Gruppie- rungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆- Alkoxy substituiert sein kann, und/oder einen über ein Stickstoffatom ge- bundenen 5- bis 7-gliedrigen heterocyclischen Rest, der weitere Hetero- atome enthalten und aromatisch sein kann, ein- oder mehrfach substitu- iert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₆-Alkyl ein- oder mehrfach substituiert sein kann;
Aryl oder Hetaryl, das durch C₁-C₁₈-Alkyl, C₁-C₆-Alkoxy, Cyano, Halo- gen, -CONHR² und/oder Aryl- oder Hetarylazo, das jeweils durch C₁-C₁₀- Alkyl, C₁-C₆-Alkoxy oder Cyano substituiert sein kann, ein- oder mehr- fach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆- Alkyl, C₁-C₆-Alkoxy, Halogen, Hydroxy, Carboxy oder Cyano substituiert sein kann.

3. Verfahren nach Anspruch 1 oder 2, **dadurch gekennzeichnet, daß** man ein aprotisches Lösungsmittel einsetzt.

4. Verfahren nach Anspruch 1 oder 2, **dadurch gekennzeichnet, daß** man ein protisches organisches Lösungsmittel einsetzt.

5. Verfahren nach den Ansprüchen 1 bis 4, **dadurch gekennzeichnet, daß** man als Base eine starke anorganische oder organische alkalimetallhaltige Base einsetzt.

6. Verfahren nach den Ansprüchen 1 bis 5, **dadurch gekennzeichnet, daß** man als Base ein Alkalimetallalkoholat einsetzt.

7. Verfahren nach den Ansprüchen 1 bis 6, **dadurch gekennzeichnet, daß** man eine stickstoffhaltige Base mit geringer nucleophiler Wirkung zusätzlich als Hilfsbase einsetzt.

## Claims

1. A process for preparing quaterrylene-3,4:13,14-tetracarboximides of the general formula I 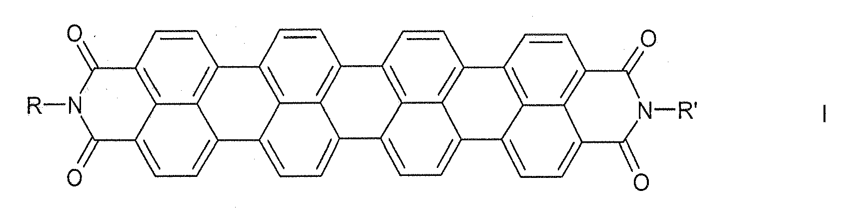 in which the variables are each defined as follows:
R, R' are each independently:
hydrogen;
(A) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by:
(i) C₁-C₁₂-alkoxy, C₁-C₆-alkylthio, -C≡CR¹, -CR¹=CR¹₂, hydroxyl, mer- capto, halogen, cyano, nitro, -NR²R³, -NR²COR³, -CONR²R³, -SO₂NR²R³, -COOR², -SO₃R², -PR²R³ and/or -POR²R³;
(ii) aryl or hetaryl, to which may be fused further saturated or unsatu- rated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysub- stituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³, -POR²R³, aryl and/or hetaryl, each of which may be substituted by C₁-C₁₈-alkyl, C₁- C₁₂-alkoxy, hydroxyl, mercapto, halogen, cyano, nitro, -NR²R³, -NR²COR³, -CONR²R³, -SO₂NR²R³, -COOR², -SO₃R², -PR²R³ and/or -POR²R³;
(iii) C₃-C₈-cycloalkyl whose carbon skeleton may be interrupted by one or more -O-, -S-, -NR¹- -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hy- droxyl, mercapto, halogen, cyano, nitro, -NR²R³, -NR²COR³, -CONR²R³, -SO₂NR²R³, -COOR², -SO₃R², -PR²R³ and/or -POR²R³;
(iv) a -U-aryl radical which may be mono- or polysubstituted by the above radicals specified as substituents for the aryl radicals (ii), where U is an -O-, -S-, -NR¹-, -CO-, -SO- or -SO₂- moiety;
(B) C₃-C₈-cycloalkyl, to which may be fused further saturated or unsatu- rated 5- to 7-membered rings whose carbon skeleton may be interrupted by one or more -0-, -S-, -NR¹-, -N=CR¹-, -CR¹=CR¹-, -CO-, -SO- and/or -SO₂- moieties, where the entire ring system may be mono- or polysub- stituted by: the (i), (ii), (iii), (iv) radicals, and/or (v) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³, -POR²R³, aryl and/or saturated or unsaturated C₄-C₇- cycloalkyl whose carbon skeleton may be interrupted by one or more -O-, -S-, -NR¹-, -N=CR¹-, -CR¹=CR¹-, -CO-, -SO- and/or -SO₂- moieties, where the aryl and cycloalkyl radicals may each be mono- or polysubsti- tuted by C₁-C₁₈-alkyl and/or the above radicals specified as substituents for alkyl;
(C) aryl or hetaryl, to which may be fused further saturated or unsatu- rated 5- to 7-membered rings whose carbon skeleton may be interrupted by one or more -O-, -S-, -NR¹-, -N=CR¹-, -C≡C-, -CR¹=CR¹-, -CO-, -SO- and/or -SO₂- moieties, where the entire ring system may be substituted by the (i), (ii), (iii), (iv), (v) radicals, and/or aryl- and/or hetarylazo, each of which may be substituted by C₁-C₁₀-alkyl, C₁-C₆-alkoxy and/or cyano;
R¹ is hydrogen or C₁-C₁₈-alkyl, where the R¹ radicals may be the same or different when they occur more than once;
R², R³ are each independently hydrogen;
C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR¹;
aryl or hetaryl, to each of which may be fused further saturated or unsaturated 5- to 7-membered rings whose carbon skeleton may be interrupted by one or more -O-, -S-, -CO- and/or -SO₂- moieties, where the entire ring system may be mono- or polysubstituted by C₁-C₁₂-alkyl and/or the above radicals specified as substituents for alkyl,
which comprises reacting a perylene-3,4-dicarboximide of the general formula IIa 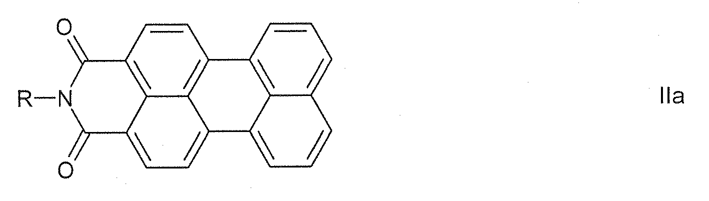 in the presence of a base-stable, high-boiling, organic solvent and of a strong alkali metal base or alkaline earth metal base, at a temperature of from 50 to 210°C, with a perylene-3,4-dicarboximide of the general formula IIb 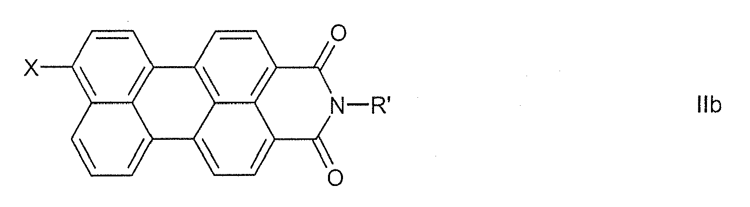 in which X is hydrogen, bromine or chlorine.

2. The process according to claim 1, which is used to prepare quaterrylene-3,4:13,14-tetracarboximides of the formula I, in which the variables are each defined as follows:
R, R' are each independently:
hydrogen;
C₁-C₃₀-alkyl whose carbon chain may be interrupted by one or more -O-, -S-, -NR¹-, -CO- and/or -SO₂- moieties and which may be mono- or poly- substituted by cyano, C₁-C₆-alkoxy, aryl which may be substituted by C₁- C₁₈-alkyl or C₁-C₆-alkoxy, and/or a 5- to 7-membered heterocyclic radical bonded via a nitrogen atom which may comprise further heteroatoms and be aromatic;
C₅-C₈-cycloalkyl whose carbon skeleton may be interrupted by one or more -O-, -S- and/or -NR¹- moieties, and/or which may be mono- or po- lysubstituted by C₁-C₆-alkyl; aryl or hetaryl which may be mono- or polysubstituted by C₁-C₁₈-alkyl, C₁-C₆-alkoxy, cyano, halogen, -CONHR² and/or aryl- or hetarylazo, each of which may be substituted by C₁-C₁₀-alkyl, C₁-C₆-alkoxy or cyano;
R¹ is hydrogen or C₁-C₆-alkyl;
R² is hydrogen; C₁-C₁₈-alkyl; aryl or hetaryl, each of which may be substi- tuted by C₁-C₆-alkyl, C₁-C₆-alkoxy, halogen, hydroxyl, carboxyl or cyano.

3. The process according to claim 1 or 2, wherein an aprotic organic solvent is used.

4. The process according to claim 1 or 2, wherein a protic organic solvent is used.

5. The process according to claims 1 to 4, wherein the base used is a strong inorganic or organic alkali metal base.

6. The process according to claims 1 to 5, wherein the base used is an alkali metal alkoxide.

7. The process according to claims 1 to 6, wherein a nitrogen base having low nucleophilic action is additionally used as an auxiliary base.

## Revendications

1. Procédé pour la préparation de quaterrylène-3,4:13,14-tétracarbodiimides de formule générale I 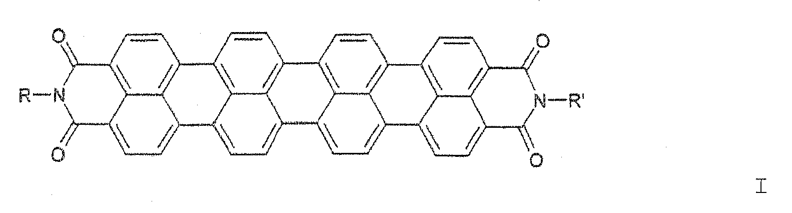 dans laquelle les variables ont les significations suivantes :
R, R' représentent, indépendamment l'un de l'autre :
un atome d'hydrogène ;
(A) un groupe alkyle en C₁-C₃₀, dont la chaîne carbonée peut être interrompue par un ou plusieurs groupements -O-, -S-, -NR¹-, -N=CR¹-, -C≡C-, -CR¹=CR¹-, -CO-, -SO- et/ou -SO₂- et qui peut être une ou plusieurs fois substitué par :
(i) alcoxy en C₁-C₁₂, alkyl(C₁-C₆)thio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³ et/ou -POR²R³ ;
(ii) aryle ou hétéroaryle, auquel peuvent être soudés d'autres cycles à 5 à 7 chaînons, saturés ou insaturés, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par : alkyle en C₁-C₁₈, alcoxy en C₁-C₁₂, alkyl(C₁-C₆)thio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³, -POR²R³, aryle et/ou hétéroaryle, pouvant chacun être substitué par alkyle en C₁-C₁₈, alcoxy en C₁-C₁₂, hydroxy, mercapto, halogène, cyano, nitro, -NR²R³, -NR²COR³, -CONR²R³, -SO₂NR²R³, -COOR², -SO₃R², -PR²R³ et/ou -POR²R³ ;
(iii) cycloalkyle en C₃-C₈, dont le squelette carboné peut être interrompu par un ou plusieurs groupements -O-, -S-, -NR¹-, -N=CR¹-, -CR¹=CR¹-, -CO-, -SO- et/ou -SO₂- et auquel peuvent être soudés d'autres cycles à 5 ou 7 chaînons, saturés ou insaturés, dont le squelette carboné peut être interrompu par un ou plusieurs groupements -O-, -S-, -NR¹-, -N=CR¹-, -CR¹=CR¹-, -CO-, -SO- et/ou -SO₂-, le système cyclique dans son ensemble pouvant être une ou plusieurs fois substitué par : alkyle en C₁-C₁₈, alcoxy en C₁-C₁₂, alkyl(C₁-C₆)thio, -C=-CR¹, -CR¹=CR¹², hydroxy, mercapto, halogène, cyano, nitro, -NR²R³, -NR²COR³, -CONR²R³, -SO₂NR²R³, -COOR², -SO₃R², -PR²R³ et/ou -POR²R³ ; (iv) un radical -U-aryle, qui peut être une ou plusieurs fois substitué par les radicaux précédents, nommés en tant que substituants pour les radicaux aryle (ii), U représentant un groupement -O-, -S-, -NR¹-, -CO-, -SO- ou -SO₂- ;
(B) un groupe cycloalkyle en C₃-C₈, auquel peuvent être soudés d'autres cycles à 5 à 7 chaînons, saturés ou insaturés, dont le squelette carboné peut être interrompu par un ou plusieurs groupements -O-, -S-, -NR¹-, -N=CR²-, -CR¹=CR¹-, -CO-, -SO- et/ou -SO₂-, le système cyclique dans son ensemble pouvant être une ou plusieurs fois substitué par : les radicaux (i), (ii), (iii), (iv) et/ou (v) alkyle en C₁-C₃₀, dont la chaîne carbonée peut être interrompue par un ou plusieurs groupements -O-, -S-, -NR¹-, -N=CR¹-, -C=C-, -CR¹=CR¹-, -CO-, -SO- et/ou -SO₂- et qui peut être une ou plusieurs fois substitué par : alcoxy en C₁-C₁₂, alkyl(C₁-C₆)thio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³, -POR²R⁵, aryle et/ou cycloalkyle en C₄-C₇ saturés ou insaturés, dont le squelette carboné peut être interrompu par un ou plusieurs groupements -O-, -S-, -NR¹-, -N=CR¹-, -CR¹=CR¹-, -CO-, -SO- et/ou -SO₂-, les radicaux aryle et cycloalkyle pouvant être une ou plusieurs fois substitués chacun par alkyle en C₁-C₁₈ et/ou par les radicaux précédents nommés en tant que substituants pour alkyle ;
(C) aryle ou hétéroaryle, auquel peuvent être soudés d'autres cycles à 5 à 7 chaînons, saturés ou insaturés, dont le squelette carboné peut être interrompu par un ou plusieurs groupements -O-, -S-, -NR¹-, -N=CR¹-, -C=C-, -CR¹=CR¹-, -CO-, -SO- et/ou -SO₂-, le système cyclique dans son ensemble pouvant être substitué par les radicaux (i), (ii), (iii), (iv), (v) et/ou aryle et/ou hétéroarylazo, chacun pouvant être substitué par alkyle en C₁-C₁₀, alcoxy en C₁-C₆ et/ou cyano ;
R¹ représente un atome d'hydrogène ou un groupe alkyle en C₁-C₁₈, les radicaux R¹, lorsqu'ils apparaissent plusieurs fois, pouvant être identiques ou différents ;
R², R³ représentent, indépendamment l'un de l'autre, un atome d'hydrogène ;
un groupe alkyle en C₁-C₁₈, dont la chaîne carbonée peut être interrompue par un ou plusieurs groupements -O-, -S-, -CO-, -SO- et/ou -SO₂- et qui peut être une ou plusieurs fois substitué par alcoxy en C₁-C₁₂, alkyl(C₁-C₆)thio, hydroxy, mercapto, halogène, cyano, nitro et/ou -COOR¹ ;
un groupe aryle ou hétéroaryle, auquel peuvent dans chaque cas être soudés d'autres cycles à 5 à 7 chaînons, saturés ou insaturés, dont le squelette carboné peut être interrompu par un ou plusieurs groupements -O-, -S-, -CO- et/ou -SO₂-, le système cyclique dans son ensemble pouvant être une ou plusieurs fois substitué par alkyle en C₁-C₁₂ et/ou par les radicaux précédents, nommés en tant que substituants pour alkyle,
**caractérisé en ce qu'**on fait réagir un pérylène-3,4-dicarboximide de formule générale IIa 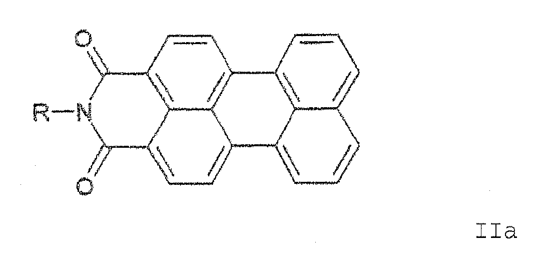 en présence d'un solvant organique à haut point d'ébullition, stable vis-à-vis des bases, et d'une base forte contenant un métal alcalin ou alcalino-terreux, à une température de 50 à 210 °C, avec un pérylène-3,4-dicarboximide de formule générale IIb 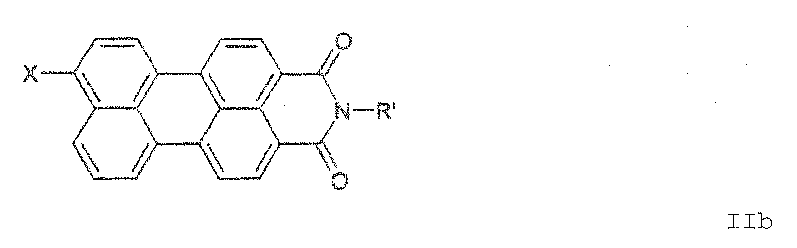 dans laquelle X représente un atome d'hydrogène, de brome ou de chlore.

2. Procédé selon la revendication 1, **caractérisé en ce qu'**on l'utilise pour la préparation de quaterrylène-3,4:13,14-tétracarbodiimides de formule I, dans laquelle les variables ont les significations suivantes :
R, R' représentent, indépendamment l'un de l'autre : un atome d'hydrogène ;
un groupe alkyle en C₁-C₃₀, dont la chaîne carbonée peut être interrompue par un ou plusieurs groupements -O-, -S-, -NR¹-, -CO- et/ou -SO₂- et qui peut être une ou plusieurs fois substitué par cyano, alcoxy en C₁-C₆, aryle, qui peut être substitué par alkyle en C₁-C₁₈ ou alcoxy en C₁-C₆, et/ou par un radical hétérocyclique à 5 à 7 chaînons, lié par un atome d'azote, qui peut être aromatique et contenir d'autres hétéroatomes ;
un groupe cycloalkyle en C₅-C₈, dont le squelette carboné peut être interrompu par un ou plusieurs groupements -O-, -S- et/ou -NR¹- et/ou qui peut être une ou plusieurs fois substitué par alkyle en C₁-C₆ ;
un groupe aryle ou hétéroaryle, qui peut être une ou plusieurs fois substitué par alkyle en C₁-C₁₈, alcoxy en C₁-C₆, cyano, halogène, -CONHR² et/ou aryle ou hétéroarylazo pouvant chacun être substitué(s) par alkyle en C₁-C₁₀, alcoxy en C₁-C₆ ou cyano ;
R¹ représente un atome d'hydrogène ou un groupe alkyle en C₁-C₆ ;
R² représente un atome d'hydrogène ; un groupe alkyle en C₁-C₁₈ ; aryle ou hétéroaryle pouvant chacun être substitué par alkyle en C₁-C₆, alcoxy en C₁-C₆, halogène, hydroxy, carboxy ou cyano.

3. Procédé selon la revendication 1 ou 2, **caractérisé en ce qu'**on utilise un solvant aprotique.

4. Procédé selon la revendication 1 ou 2, **caractérisé en ce qu'**on utilise un solvant organique protique.

5. Procédé selon les revendications 1 à 4, **caractérisé en ce qu'**on utilise comme base une base forte inorganique ou organique contenant un métal alcalin.

6. Procédé selon les revendications 1 à 5, **caractérisé en ce qu'**on utilise comme base un alcoolate de métal alcalin.

7. Procédé selon les revendications 1 à 6, **caractérisé en ce qu'**on utilise comme base auxiliaire une base azotée à faible action nucléophile.
